(19) Europäisches Patentamt
European Patent Office
Office européen des brevets

(11) EP 2 782 547 B1

(12) **EUROPEAN PATENT SPECIFICATION**

(45) Date of publication and mention of the grant of the patent:
**31.08.2016 Bulletin 2016/35**

(21) Application number: **12808498.5**

(22) Date of filing: **22.11.2012**

(51) Int Cl.:
*A61K 8/06* (2006.01)     *A61K 8/25* (2006.01)
*A61K 8/31* (2006.01)     *A61K 8/34* (2006.01)
*A61K 8/39* (2006.01)     *A61Q 13/00* (2006.01)
*A61Q 15/00* (2006.01)     *A61K 8/86* (2006.01)

(86) International application number:
**PCT/IB2012/056618**

(87) International publication number:
**WO 2013/076675 (30.05.2013 Gazette 2013/22)**

(54) **FRAGRANCING COMPOSITION OF PICKERING EMULSION TYPE**

PICKERING EMULSION ALS DUFTSTOFFZUSAMMENSETZUNG

COMPOSITION PARFUMANTE DE TYPE EMULSION PICKERING

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2011 FR 1160797**
**25.11.2011 FR 1160791**
**07.12.2011 US 201161567700 P**
**07.12.2011 US 201161567678 P**

(43) Date of publication of application:
**01.10.2014 Bulletin 2014/40**

(73) Proprietor: **L'Oréal**
**75008 Paris (FR)**

(72) Inventor: **BARA, Isabelle**
**94210 La Varenne Saint Hilaire (FR)**

(74) Representative: **Nony**
**11 rue Saint-Georges**
**75009 Paris (FR)**

(56) References cited:
**FR-A1- 2 208 642     US-A- 4 185 086**

## Description

The present invention relates to the field of fragrances, and especially to the field of compositions in the form of beads in suspension in a liquid, which are used after shaking.

The invention relates more particularly to a composition in the form of an oil-in-water emulsion, comprising, in a cosmetically acceptable medium:

(i) a dispersed oily phase comprising at least one apolar hydrocarbon-based oil;
(ii) a continuous aqueous-alcoholic phase comprising at least one $C_1$-$C_4$ monoalcohol;
(iii) at least 1% by weight of fragrancing substance(s), relative to the total weight of the said composition;
(iv) magnesium silicate particles; and
(v) at least one polyalkylene glycol.

The invention also relates to a process for fragrancing human keratin materials and/or clothing in contact with said keratin materials, comprising the application to these keratin materials and/or to said clothing of a composition in accordance with the invention.

Whether it is a case of masking a strong and/or unpleasant odour or of giving a pleasant odour, people have always sought to fragrance themselves and to fragrance the objects or places surrounding them.

This is why a very large number of new fragrances appear on the market every year, namely about 500 new product launches in Europe per year.

Very few of these fragrances finally prove to be successful on the perfumery market, for many reasons. One of these reasons is the appeal of the product presentation.

Specifically, in order to demark themselves from their competitors, perfumers may present their fragrances in luxury bottles, or even give them evocative names, in order to attract the consumer.

In addition or as an alternative to the presentation of fragrances in a luxury bottle, a particular galenical form of fragrance which, by its very nature, draws the consumer's attention may be proposed.

Two-phase compositions which appeal to consumers on account of their esthetic nature are known. These compositions consist of two mutually immiscible phases, which are mixed together extemporaneously by shaking before use

Unlike the compositions of the present invention, the two phases of these compositions are two separate superposed layers of different color, not forming any particular pattern, and separated by a quite distinct interface.

Patent application FR 2 208 642 teaches the use and preparation, in general, of two-phase liquid cosmetic compositions, called of Pickering emulsion type, which are appealing due to the fact that the oily phase is dispersed in the form of spheres in a homogeneous mixture between an organic liquid and water. Finely divided solid particles are adsorbed at the interface between the oil and the homogeneous mixture, and serve to stabilize the oil spheres.

However, said document never refers to fragrancing compositions.

Patent EP 1 005 849 for its part discloses two-phase compositions for treating the hair or the scalp.

There is a need for alternative fragrancing compositions or compositions that are improved over the known fragrancing compositions, especially aqueous-alcoholic solutions and two-phase compositions.

In the context of the present invention, the inventors have demonstrated that the solid particles located at the interface of the two phases in these compositions have a tendency to sediment over time when they comprise high contents of fragrancing substance(s), namely amounts of greater than or equal to 1% by weight relative to the total weight of the composition. They have also discovered that when the content of fragrancing substance(s) reaches 5% by weight, the emulsion becomes destabilized and so-called phase separation takes place.

There is thus a need for a composition of "Pickering" emulsion type with fragrancing properties which can comprise a content of fragrancing substance(s) of greater than or equal to 1% by weight relative to the total weight of the composition, while at the same time maintaining good stability over time.

The object of the present invention is to satisfy this need.

The inventors have demonstrated, unexpectedly, in the context of the present patent application, that the addition of polyalkylene glycols to a composition of "Pickering" emulsion type comprising a content of fragrancing substance(s) of greater than or equal to 1% makes it possible to prevent the adverse phenomena indicated previously, namely sedimentation or phase separation.

Thus, a subject of the present invention is firstly a liquid composition in the form of an oil-in-water emulsion, comprising, in a cosmetically acceptable medium:

(i) a dispersed oily phase comprising at least one apolar hydrocarbon-based oil;
(ii) a continuous aqueous-alcoholic phase comprising at least one $C_1$-$C_4$ monoalcohol;
iii) at least 1% by weight of fragrancing substance(s), relative to the total weight of the said composition;

(iv) magnesium silicate particles; and
(v) at least one polyalkylene glycol.

To the inventors' knowledge, the problem posed in the present invention has never been raised, and in consequence solving this problem by adding polyalkylene glycol has never been proposed.

The term "fragrancing substance" means any fragrance or aroma that is capable of giving off a pleasant odour.

The term "cosmetically acceptable medium" means a medium that is compatible with the skin and/or its integuments or mucous membranes, that has a pleasant color, odor and feel and that does not cause any unacceptable discomfort (stinging, tautness or redness) liable to dissuade the consumer from using this composition.

The term "human keratin materials" means the skin (of the body or face or around the eyes), hair, eyelashes, eyebrows, bodily hair, nails, lips and mucous membranes, and more particularly the skin.

The term "aqueous-alcoholic phase" means a phase comprising at least water and an organic compound comprising at least one OH function.

The present invention consequently provides a composition with fragrancing properties due to the presence of a content of fragrancing substance(s) that may be greater than or equal to 1% by weight relative to the total weight of the composition while at the same time maintaining good stability over time.

On account of their pearl suspension appearance, the compositions according to the invention are particularly appealing, especially when compared with conventional fragrancing compositions of aqueous-alcoholic or two-phase nature.

The compositions in accordance with the invention also have the advantage of not needing to use surfactants. The absence of these compounds makes it possible to overcome a certain number of drawbacks, such as those mentioned in patent application FR 2 208 642.

The compositions in accordance with the invention afford, when compared with the usual galenical forms of fragrances, especially aqueous-alcoholic solutions, a substantial moisturizing effect and also better sensory effects such as a more silky and less dry feel on the skin.

The compositions in accordance with the invention may thus be readily prepared without heating or at room temperature, in contrast with the known techniques for manufacturing emulsions, which are generally prepared with heating. Mention may be made especially of the production of emulsions by simple stirring, for example using a paddle blender.

In certain embodiments, a composition in accordance with the invention may also be colored. In this embodiment, a composition according to the invention may comprise, either in the lipophilic dispersed phase or in the hydrophilic continuous phase, or in each of the two phases, at least one coloring agent.

In the rest of the description, (i) a colorant present in the dispersed lipophilic phase is denoted as a "first colorant" and (ii) a colorant present in the continuous hydrophilic phase is denoted as a "second colorant", including when only one from among the dispersed and continuous phases comprises a colorant.

In other embodiments, a composition according to the invention comprises at least one liposoluble first colorant in the dispersed oily phase (i) and at least one water-soluble second colorant in the continuous aqueous-alcoholic phase (ii).

Advantageously, said first and second colorants give the dispersed and continuous phases of a composition according to the invention shades that differ from each other.

Specifically, the addition of at least one colorant to each of the phases of a composition of Pickering type according to the invention so as advantageously to give these phases different shades makes it possible, after correct shaking, to obtain a third shade that is different from the two preceding ones.

The colorant(s) present, respectively, (i) in the dispersed oily phase and (ii) in the continuous aqueous-alcoholic phase thus constitute homogeneity markers that enable the user to determine simply and directly if the composition according to the invention is sufficiently homogenized to be able to be used.

For the purposes of the invention, two shades are considered as being different from each other when the difference between their respective colors can be distinguished by the user with the naked eye.

In particular, the difference between shades ($\Delta$E) can be measured in the $L^*$ to $b^*$ colorimetric measuring system as defined according to the CIE 1976 standard.

The value of $\Delta$E is calculated according to formula (I) below: $\triangle E = \sqrt{(L_1-L_2)^2+(a_1-a_2)^2+(b_1-b_2)^2}$, in which:

$L_1$, $a_1$, $b_1$ are the CIE Lab colorimetric space coordinates of the first color to be compared, and
$L_2$, $a_2$, $b_2$ are the CIE Lab colorimetric space coordinates of the second color to be compared.

The present invention is also directed toward a process for fragrancing human keratin materials and/or clothing that is in contact with said keratin materials, comprising the application to said keratin materials and/or said clothing of a composition in accordance with the invention.

For the purposes of the present invention, the term "prevent" means reducing the risk of occurrence of the phenomena more particularly discussed previously, namely the sedimentation of the interface particles or even the phase separation of the compounds of the composition according to the invention.

Preferentially, the composition of the invention will be in liquid form.

For the purposes of the invention, the term "liquid composition" means a composition that is not in solid form and whose viscosity, measured using a Rheomat 180 viscometer at 25°C at a spin speed of 200 rpm after 10 minutes of rotation, is less than or equal to 2 Pa.s and more preferentially ranges from 0.01 Pa.s to 0.5 Pa.s.

For the purposes of the present invention, the term "surfactant" means an amphiphilic molecule, i.e. a molecule that has two parts of different polarity, one being lipophilic (which retains fatty substances) and apolar, and the other hydrophilic (water-miscible) and polar. Thus, the solid particles in accordance with the invention are not considered as surfactants for the purposes of the present invention.

**Dispersed oily phase**

As indicated previously, a composition in accordance with the invention comprises a dispersed oily phase comprising at least one apolar hydrocarbon-based oil.

For the purposes of the present invention, the term "apolar oil" means an oil whose solubility parameter at 25°C, $\delta a$, is equal to 0 $(J/cm^3)^{1/2}$.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:

- $\delta D$ characterizes the London dispersion forces derived from the formation of dipoles induced during molecular impacts;
- $\delta p$ characterizes the Debye interaction forces between permanent dipoles and also the Keesom interaction forces between induced dipoles and permanent dipoles;
- $\delta h$ characterizes the specific interaction forces (such as hydrogen bonding, acid/base, donor/acceptor, etc.); and
- $\delta a$ is determined by the equation: $\delta a = (\delta p^2 + \delta h^2)^{1/2}$.

The parameters $\delta p$, $\delta h$, $\delta D$ and $\delta a$ are expressed in $(J/cm^3)^{1/2}$.

An apolar oil in accordance with the invention is hydrocarbon-based.

The term "hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

According to one embodiment, the apolar hydrocarbon-based oil according to the invention is free of heteroatoms. The term "heteroatom" means an atom other than carbon or hydrogen.

The apolar oil according to the invention may be nonvolatile.

The term "nonvolatile oil" means any oil whose vapor pressure at room temperature and atmospheric pressure is nonzero and less than 0.02 mmHg and better still less than $10^{-3}$ mmHg.

An apolar hydrocarbon-based oil in accordance with the invention advantageously represents from 5% to 40% by weight relative to the total weight of the composition, preferably from 5% to 30% by weight and preferentially from 10% to 30% by weight relative to the total weight of the composition containing it.

According to one preferred embodiment, the apolar hydrocarbon-based oil used in the present invention is nonvolatile and may be chosen advantageously from linear or branched saturated alkanes.

An apolar hydrocarbon-based oil according to the invention may be chosen from oils whose molecular mass is between 300 and 900 g/mol and preferably between 350 and 800 g/mol.

According to one preferred embodiment, the apolar hydrocarbon-based oil is chosen from linear or branched hydrocarbons of mineral or synthetic origin, preferably from a volatile or nonvolatile liquid paraffin oil, hydrogenated isoparaffin, naphthalene oil, a totally or partially hydrogenated liquid polydecene, isoeicosane, a decene/butene copolymer, or a polybutene/polyisobutene copolymer, and mixtures thereof.

The term "hydrocarbon" means a compound consisting of carbon and hydrogen.

According to one particular embodiment, the following will preferably be used among the apolar hydrocarbon-based oils:

- hydrogenated isoparaffins, for instance the hydrogenated polyisobutene sold under the name Parleam by the company Rossow, or under the name Polysynlane by Nippon Oil & Fat or Polyester Corp.;

- the $C_8$-$C_9$ isoparaffin sold under the name Isopar E by ExxonMobil Chemical;
- the $C_{11}$-$C_{13}$ isoparaffin from Exxon sold under the name Isopar L; or
- the $C_{13}$-$C_{14}$ isoparaffin sold under the name Isopar M by the company ExxonMobil Chemical;
- the liquid paraffins from Petro Canada: Puretol 9, or Blandol from Sonneborn, or Marcol 82 sold by ExxonMobil Chemical, and
- the plant perhydrosqualenes sold under the name Olive Squalane by SOS Corporation Alimentaria, or Vegetable Squalane by Lake Oil, or Exolive by Caroi Line Cosmetica.

A dispersed oily phase according to the invention is in the form of spheres of more or less homogeneous size, which may range between 0.5 and 20 mm, preferably between 0.5 and 10 mm and preferentially between 1 and 5 mm.

The number of oil spheres will vary as a function of the percentage of fragrance, oil and magnesium silicate particles. From 1 to several million per liter of formulation may be counted as a function of their size. These spheres may be present in the upper or lower part of the composition comprising them or in both parts of the composition.

As indicated previously, a dispersed oily phase according to the invention may also comprise at least one first liposoluble coloring agent.

Such a colorant may be of natural or synthetic origin.

For the purposes of the invention, the term "liposoluble colorant" means any generally organic, natural or synthetic compound, which is soluble in an oily phase.

Examples of liposoluble colorants in accordance with the invention that may be mentioned include:

- a violet organic colorant, D&C Violet No. 2 K7014 Chemical name: Alizurol purple SS;
- a green organic colorant, D&C Green No. 6 K7016 / 90097 D&C Green 6 Chemical name: quinizarin green E SS;
- a pink organic colorant, D&C Red No. 21 K7061 / Suncroma D&C Red 21 C 14-032 Chemical name: Eosin; and
- an orange vegetable colorant, Betatene 30% OLV Chemical name: Carotenoids (CI. 75130-E160 A) at 30% in olive oil.

The liposoluble colorants in accordance with the invention may also be chosen from Sudan Red, D&C Red 17, β-carotene, Sudan Brown, D&C Yellow 11, D&C Orange 5, quinoline yellow and annatto.

The dispersed oily phase according to the invention may also comprise other oils, such as silicone oils or plant oils, provided that they do not impair the stability of the composition according to the invention.

## Continuous aqueous-alcoholic phase

As indicated previously, a composition in accordance with the invention comprises, in addition to a dispersed oily phase, a continuous aqueous phase comprising at least one $C_1$-$C_4$ monoalcohol.

A monoalcohol in accordance with the invention may preferably be chosen from linear $C_1$-$C_4$ hydroxyalkyls. Ethanol will be used more particularly.

Such a monoalcohol may be present in a content ranging from 50% to 90% by weight and preferably from 53% to 70% by weight relative to the total weight of the composition.

This continuous aqueous phase comprises at least water. This water is then preferably present in a content ranging from 0.5% to 10% by weight and more preferentially from 1% to 5% by weight relative to the total weight of the composition according to the invention.

As indicated previously, the continuous aqueous-alcoholic phase in accordance with the invention may also comprise at least a second liposoluble colorant. This colorant may be of synthetic or plant origin.

For the purposes of the invention, the term "water-soluble colorant" means any natural or synthetic, generally organic compound, which is soluble in an aqueous phase or in water-miscible solvents such as $C_1$-$C_4$ monoalcohols and which is capable of coloring.

Mention may in particular be made, as water-soluble dyes suitable for the invention, of synthetic or natural water-soluble dyes, such as, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (oenocyanin, black carrot, hibiscus, elder), caramel and riboflavin.

According to one particularly preferred form of the invention, the dispersed oily phase and the continuous aqueous phase are adjusted such that the absolute value of the difference in density (Δd) between the dispersed oily phase and the continuous aqueous phase is not more than 0.05 and advantageously not more than 0.01.

## Magnesium silicate particles

The composition according to the invention also comprises magnesium silicate particles for stabilizing the composition according to the invention by positioning themselves at the dispersed phase/continuous phase interface.

The magnesium silicates in accordance with the invention may be of natural or synthetic origin.

Talc is particularly preferred as a magnesium silicate in accordance with the invention.

Talcs are hydrated magnesium silicates usually comprising aluminium silicate. The crystal structure of talc consists of repeating layers of a sandwich of brucite between layers of silica.

The talc in accordance with the invention may be chosen more particularly from those sold under the names Rose Talc® and Talc SG-2000® sold by the company Nippon Talc, Luzenac Pharma M® sold by the company Luzenac, J-68BC from US Corporation and Micro ACE-P-3® sold by the company Nippon Talc.

The solid magnesium silicate particles may be used in a content preferably of between 0.05% and 5% by weight, preferentially between 0.1% and 2% by weight and even more preferentially between 0.1% and 1% by weight relative to the total weight of the composition containing them.

They also preferably have a size of between 1 and 30 $\mu$m, preferentially between 1 and 20 $\mu$m and more preferentially between 2 and 15 $\mu$m.

**Fragrancing substances**

A composition in accordance with the invention also comprises at least 1% by weight of fragrancing substance(s), or perfume(s), relative to the total weight of the composition.

Perfumes are compositions especially containing the starting materials described in S. Arctander, Perfume and Flavor Chemicals (Montclair, N.J., 1969), in S. Arctander, Perfume and Flavor Materials of Natural Origin (Elizabeth, N.J., 1960) and in Flavor and Fragrance Materials - 1991, Allured Publishing Co., Wheaton, III.

They may also be natural products, for instance essential oils, absolutes, resinoids, resins, concretes, and/or synthetic products (terpene or sesquiterpene hydrocarbons, alcohols, phenols, aldehydes, ketones, ethers, acids, esters, nitriles or peroxides, which may be saturated or unsaturated, and aliphatic or cyclic).

According to the definition given in international standard ISO 9235 and adopted by the Commission of the European Pharmacopoeia, an essential oil is an odorous product generally of complex composition, obtained from a botanically defined plant raw material, either by steam entrainment, or by dry distillation, or via an appropriate mechanical process without heating (cold pressing). The essential oil is usually separated from the aqueous phase via a physical process that does not result in any significant change in the composition.

Among the essential oils that may be used according to the invention, mention may be made of those obtained from plants belonging to the following botanical families:

Abietaceae or Pinaceae: conifers; Amaryllidaceae; Anacardaceae; Anonaceae:

ylang ylang; Apiaceae (for example Umbelliferae): dill, angelica, coriander, sea fennel, carrot, parsley; Araceae; Aristolochiaceae; Asteraceae: yarrow, artemisia, camomile, helichrysum; Betulaceae; Brassicaceae; Burseraceae: frankincense; Carophyllaceae; Canellaceae; Cesalpiniaceae: copaifera (copaiba balsam); Chenopodaceae; Cistaceae: rock rose; Cyperaceae; Dipterocarpaceae; Ericaceae: gaultheria (wintergreen); Euphorbiaceae; Fabaceae; Geraniaceae: geranium; Guttiferae; Hamamelidaceae; Hernandiaceae; Hypericaceae: St-John's wort; Iridaceae; Juglandaceae; Lamiaceae: thyme, oregano, monarda, savory, basil, marjorams, mints, patchouli, lavenders, sages, catnip, rosemary, hyssop, balm; Lauraceae: ravensara, sweet bay, rosewood, cinnamon, litsea; Liliaceae: garlic; Magnoliaceae: magnolia; Malvaceae; Meliaceae; Monimiaceae; Moraceae: hemp, hop; Myricaceae; Myristicaceae: nutmeg; Myrtaceae: eucalyptus, tea tree, paperbark tree, cajuput, backhousia, clove, myrtle; Oleaceae; Piperaceae: pepper; Pittosporaceae; Poaceae: lemon balm, lemongrass, vetiver; Polygonaceae; Renonculaceae; Rosaceae: roses; Rubiaceae; Rutaceae: all citrus plants; Salicaceae; Santalaceae: sandalwood; Saxifragaceae; Schisandraceae; Styracaceae: benjoin; Thymelaceae: agar wood; Tilliaceae; Valerianaceae: valerian, spikenard; Verbenaceae: lantana, verbena; Violaceae; Zingiberaceae: galangal, turmeric, cardamom, ginger; Zygophyllaceae.

Mention may also be made of the essential oils extracted from flowers (lily, lavender, rose, jasmine, ylang ylang, neroli), from stems and leaves (patchouli, geranium, petitgrain), from fruit (coriander, aniseed, cumin, juniper), from fruit peel (bergamot, lemon, orange), from roots (angelica, celery, cardamom, iris, rattan palm, ginger), from wood (pinewood, sandalwood, gaiac wood, rose of cedar, camphor), from grasses and gramineae (tarragon, rosemary, basil, lemongrass, sage, thyme), from needles and branches (spruce, fir, pine, dwarf pine) and from resins and balms (galbanum, elemi, benjoin, myrrh, olibanum, opopanax).

Examples of fragrancing substances are especially: geraniol, geranyl acetate, farnesol, borneol, bornyl acetate, linolool, linalyl acetate, linalyl propionate, linalyl butyrate, tetrahydrolinolool, citronellol, citronellyl acetate, citronellyl formate, citronellyl propionate, dihydromyrcenol, dihydromyrcenyl acetate, tetrahydromyrcenol, terpineol, terpinyl acetate, nopol, nopyl acetate, nerol, neryl acetate, 2-phenylethanol, 2-phenylethyl acetate, benzyl alcohol, benzyl acetate, benzyl salicylate, styrallyl acetate, benzyl benzoate, amyl salicylate, dimethylbenzylcarbinol, trichloromethylphenylcarbinyl acetate, p-tert-butylcyclohexyl acetate, isononyl acetate, vetiveryl acetate, vetiverol, $\alpha$-hexylcinnamaldehyde, 2-methyl-3-(p-tert-butylphenyl)propanal, 2-methyl-3-(p-isopropylphenyl)propanal, 3-(p-tert-butylphenyl)propanal, 2,4-dimethylcyclohex-3-enylcarboxaldehyde, tricyclodecenyl acetate, tricyclodecenyl propionate, 4-(4-hydroxy-4-methylpentyl)-3-cyclohexenecarboxaldehyde, 4-(4-methyl-3-pentenyl)-3-cyclohexenecarboxaldehyde, 4-acetoxy-3-pentyltetrahydropyran, 3-carboxymethyl-2-pentylcyclopentane, 2-n-4-heptylcyclopentanone, 3-methyl-2-pentyl-2-cyclopentenone, menthone, carvone, tagetone, geranylacetone, n-decanal, n-dodecanal, 9-decen-1-ol, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepinonitrile, aubepine, heliotropin, coumarin, eugenol, vanillin, diphenyl ether, citral, citronellal, hydroxycitronellal, damascone, ionones, methylionones, isomethylionones, solanone, irones, cis-3-hexenol and esters thereof, musk-indans, musk-tetralins, musk-isochromans, macrocyclic ketones, musk-macrolactones, aliphatic musks, ethylene brassylate and rose essence, and mixtures thereof.

According to one preferred embodiment of the invention, a mixture of different fragrancing substances that generate in common a note that is pleasant to the user is used.

The fragrancing substances will preferably be chosen such that they produce notes (head, heart and base) in the following families: citrine, aromatic, floral, spicy, woody, gourmand, chypre, fougere, leathery, musk.

The fragrancing compositions of the invention must contain at least 1% by weight of fragrancing substance(s), preferably at most 30% by weight, in particular from 1% to 25% by weight and especially from 3% to 15% by weight of fragrancing substance(s) relative to the total weight of the composition comprising them.

## Polyalkylene glycol

A composition in accordance with the invention also comprises at least one polyalkylene glycol.

The presence of such a compound in the fragrancing compositions in accordance with the invention advantageously makes it possible to incorporate an amount of greater than or equal to 1% by weight of fragrancing substances into a composition of Pickering type in accordance with the invention, without observing any sedimentation and/or phase separation generally observed when the content of fragrancing substances reaches such proportions in such compositions.

According to one particular embodiment, a polyalkylene glycol in accordance with the invention may be of formula (I) below:

$$\text{H-[O-R-]}_n\text{-OH} \qquad (I),$$

in which

- R represents a linear alkyl chain containing from 1 to 4 carbon atoms, and
- n is an integer ranging from 4 to 200 and advantageously from 4 to 40.

According to one particular embodiment, n is an integer ranging from 4 to 20 and advantageously from 6 to 10.

According to one particular embodiment, a polyalkylene glycol in accordance with the invention is a polyethylene glycol.

Thus, according to one preferred embodiment, a polyalkylene glycol in accordance with the invention is a polyethylene glycol with n being an integer ranging from 4 to 20 and advantageously from 6 to 10.

According to one preferred embodiment, a polyalkylene glycol in accordance with the invention may be chosen from PEG-8OE such as the product sold under the trade name Polyethylene Glycol 400 DUB PEG-8 from the company Stéarineries Dubois or under the trade name Polyglycol 400 from the company Clariant, or alternatively PEG 6OE sold under the trade name Carbowax PEG-300 from the company Dow chemical.

The polyalkylene glycol(s) are preferably present in the composition in concentrations ranging from 0.1% to 3% and more preferentially from 0.5% to 2% by weight relative to the total weight of the composition.

## Water-soluble metal salt

A composition in accordance with the invention may also comprise at least one water-soluble metal salt.

The term "water-soluble metal salt" means any cosmetic or dermatological metal salt that can be completely dissolved in molecular form in a liquid aqueous phase comprising water or a mixture of water/$C_1$-$C_4$ monoalcohol.

According to the present invention, the term "metal salt" means a salt of a metal, i.e. of a simple substance that is capable of releasing simple cations (Dictionnaire de la Chimie et de ses Applications, Duval & Duval, 3rd Edition, 1978, Technique et Documentation).

The water-soluble metal salts are more particularly chosen from water-soluble salts of alkali metals or of alkaline-earth metals.

As water-soluble salts of alkali metals that are useful according to the invention, mention may be made in particular of sodium or potassium salts.

As water-soluble salts of alkaline-earth metals that are useful according to the invention, mention may be made in particular of magnesium or calcium salts.

These salts may be, for example, carbonates, bicarbonates, sulfates, chlorides, nitrates, acetates or hydroxides, and also salts of $\alpha$-hydroxy acids or salts of fruit acids (citrate, tartrate, lactate or malate), or alternatively salts of amino acids (aspartate, arginate, glucocholate or fumarate).

Preferably, a salt according to the invention is chosen from calcium chloride and sodium chloride. It is preferably calcium chloride.

The water-soluble metal salts in accordance with the invention may be present in a content of between 0% and 5% by weight and preferably between 0.2% and 1% by weight relative to the total weight of the composition.

## Additives

The composition of the invention may also comprise any additive usually used in the field of fragrances, chosen especially from antioxidants, cosmetic or dermatological active agents, for instance emollients or softeners such as sweet almond oil or apricot kernel oil, moisturizers such as glycerol, calmatives such as $\alpha$-bisabolol, allantoin or *Aloe vera*; vitamins, essential fatty acids, insect repellents, propellants, peptizers, UV-screening agents, stabilizers or preserving agents, gellants or thickeners, nacres and glitter flakes, and mixtures thereof. When they are present in the composition of the invention, these additives may be present in an amount ranging from 0.001% to 10% and better still from 0.01% to 5% by weight relative to the total weight of the composition.

Among the antioxidants, examples that may be mentioned include BHA (*tert*-butyl-4-hydroxyanisole), BHT (2,6-di-*tert*-butyl-p-cresol), tocopherols such as vitamin E and derivatives thereof such as tocopheryl acetate.

## Conditioning

A composition according to the invention may constitute a fragrancing composition and may especially be in the form of a spray or aerosol (body mist or body splash), an eau fraiche, an eau de toilette, an eau de parfum or an aftershave lotion.

Preferably, it constitutes a fragrancing composition. A composition in accordance with the invention may, for example, be conditioned in a bottle.

A composition of the invention may be dispensed by means of various systems that are well known to those skilled in the art, such as sprayers with or without pressurized gas, or alternatively roll-on applicators.

A composition according to the invention may be manufactured according to the known processes of Pickering emulsions.

The compositions according to the invention may also be applied in the form of fine particles by means of pressurization devices. The devices in accordance with the invention are well known to those skilled in the art and comprise non-aerosol pumps or "atomizers", piezoelectric devices, aerosol containers comprising a propellant and also aerosol pumps using compressed air as propellant. These devices are especially described in patents US 4 077 441 and US 4 850 517 (which form an integral part of the content of the description), more particularly relating to fragrancing compositions.

The compositions conditioned as aerosols in accordance with the invention generally contain conventional propellants, for instance dimethyl ether, isobutane, n-butane, propane or trichlorofluoromethane.

As indicated previously, a composition of Pickering emulsion type in accordance with the invention must be shaken before use so as to make the composition perfectly homogeneous.

In the description and the examples that follow, unless otherwise mentioned, the percentages are weight percentages and the ranges of values written in the form "between ... and ..." include the stated lower and upper limits. The ingredients are mixed, before being formed, in the order and under conditions that may readily be determined by a person skilled in the art.

The examples below are presented as non-limiting illustrations of the field of the invention.

## EXAMPLES

### Example 1: Composition containing 14% fragrance

| Compounds | % by weight relative to the total weight of the composition |
|---|---|
| Water | 3 |
| CaCl$_2$ | 0.75 |
| PEG 8OE | 1 |
| Talc | 0.3 |
| Hydrogenated isoparaffin | 25 |
| Fragrance * | 14 |
| Ethylhexyl methoxycinnamate (and) ethylhexyl salicylate (and) butyl methoxydibenzoylmethane (Covabsorb® Sensient) liposoluble organic UV-screening agent | 1.5 |
| BHT (butylated hydroxytoluene) | 0.04 |
| D&C Green 5 | 0.04 |
| Ethanol | qs 100 |
| * the fragrance used comprises 40% hedione, 15% geraniol, 22% linalyl acetate, 2% ethylvanillin, 1% Ambrox DL, 10% rose essence, 5% citral and 5% patchoulol. | |

The difference in density between the two phases of the composition is 0.00635.

The composition thus obtained consists of a two-phase solution containing beads suspended in a perfectly clear blue-colored liquid.

The beads are distributed at the bottom of the bottle comprising the composition, their size being from 1 to 5 mm.

After moderate manual shaking for 5 seconds, the composition becomes readily homogenized and may be vaporized uniformly, for example using a standard pump-action bottle.

The result on the skin is a fragranced fluid deposit, which feels fresh on application, and after drying has a silky feel on the skin.

Once the bottle has been left to stand, the original two-phase distribution of the composition is regained after 5 minutes.

After two months, the appearance and the organoleptic qualities of the product are unchanged, both at room temperature and at 37°C.

### Examples 2 and 3: Compositions containing 25% fragrance with or without polyalkylene glycol

| Compounds | Example 2 (invention) | Example 3 (outside invention) |
|---|---|---|
| Water | 1 | 1 |
| CaCl$_2$ | 0.5 | 0.5 |
| PEG 8OE | 2 | 0 |
| Talc | 0.5 | 0.5 |
| Hydrogenated isoparaffin | 15 | 15 |
| Fragrance * | 25 | 25 |
| Ethylhexyl methoxycinnamate (and) ethylhexyl salicylate (and) butyl methoxydibenzoylmethane (Covabsorb® Sensient) Liposoluble organic UV-screening agent | 1.5 | 1.5 |
| BHT (butylated hydroxytoluene) | 0.04 | 0.04 |
| D&C Green 5 | 0.04 | 0.04 |

(continued)

| Compounds | Example 2 (invention) | Example 3 (outside invention) |
|---|---|---|
| Ethanol | qs 100 | qs 100 |

* the fragrance used comprises 40% hedione, 15% geraniol, 22% linalyl acetate, 2% ethylvanillin, 1% Ambrox DL, 10% rose essence, 5% citral and 5% patchoulol.

The difference in density between the two phases of composition 2 is 0.00033.

Compositions 2 and 3 thus obtained consist of a two-phase solution containing beads suspended in a perfectly clear blue-colored liquid.

The beads are distributed at the bottom and top of the bottle comprising the composition, their heterogeneous size being from 0.5 to 10 mm.

After moderate manual shaking for 5 seconds, the compositions become readily homogenized and may be vaporized uniformly, for example using a standard pump-action bottle.

The result on the skin is a fragranced fluid deposit, which feels fresh on application, and after drying has a silky feel on the skin.

Once the bottle has been left to stand, the original two-phase distribution of the composition is regained after 5 minutes.

After two months, the appearance and the organoleptic qualities of composition 2 are unchanged, both at room temperature and at 37°C. On the other hand, composition 3 not containing any polyalkylene glycol shows coalescence of the beads after a few days.

### Example 4: Stable composition containing 1% fragrance

| Compounds | % by weight relative to the total weight of the composition |
|---|---|
| Water | 3 |
| CaCl$_2$ | 0.75 |
| PEG 8OE | 1 |
| Talc | 0.3 |
| Hydrogenated isoparaffin | 25 |
| Fragrance * | 1 |
| Ethylhexyl methoxycinnamate (and) ethylhexyl salicylate (and) butyl methoxydibenzoylmethane (Covabsorb® Sensient) liposoluble organic UV-screening agent | 1.5 |
| BHT (butylated hydroxytoluene) | 0.04 |
| Beta-carotene (orange liposoluble colorant) | $7 \times 10^{-4}$ |
| Disodium salt of fuchsin (red watersoluble colorant) | $2.7 \times 10^{-4}$ |
| Ethanol | qs 100 |

* the fragrance used comprises 40% hedione, 15% geraniol, 22% linalyl acetate, 2% ethylvanillin, 1% Ambrox DL, 10% rose essence, 5% citral and 5% patchoulol.

The difference in density between the two phases of the composition is 0.00635.

The composition thus obtained consists of a two-phase solution containing orange-colored transparent beads suspended in a perfectly clear pink-colored liquid.

The beads are distributed at the bottom of the bottle comprising the composition, their size being from 1 to 5 mm.

After moderate manual shaking for 5 seconds, the orange/pink two-colored composition becomes readily homogenized as a pinkish apricot solution, and may be vaporized uniformly, using a standard pump-action bottle.

The result on the skin is a fragranced fluid deposit, which feels fresh on application, and after drying has a silky feel on the skin.

Once the bottle has been left to stand, the original two-phase distribution of the composition is regained after 5 minutes.

After two months, the appearance and the organoleptic qualities of the product are unchanged, both at room temperature and at 37°C.

**Examples 5: Stable compositions containing 2% fragrance**

| Compounds | % by weight relative to the total weight of the composition |
|---|---|
| Water | 1 |
| $CaCl_2$ | 0.5 |
| PEG 8OE | 2 |
| Talc | 0.5 |
| Hydrogenated isoparaffin | 15 |
| Fragrance * | 2 |
| Ethylhexyl methoxycinnamate (and) ethylhexyl salicylate (and) butyl methoxydibenzoylmethane (Covabsorb® Sensient) (Liposoluble organic UV-screening agent) | 1.5 |
| BHT | 0.04 |
| D&C Violet 2 Violet liposoluble colorant | $4 \times 10^{-5}$ |
| D&C Green 5 Blue watersoluble colorant | $5 \times 10^{-4}$ |
| Ethanol | qs 100 |
| * the fragrance used comprises 40% hedione, 15% geraniol, 22% linalyl acetate, 2% ethylvanillin, 1% Ambrox DL, 10% rose essence, 5% citral and 5% patchoulol. | |

The difference in density between the two phases of the composition is 0.00033.

The composition thus obtained consist of a two-phase solution containing violet-colored beads suspended in a perfectly clear blue-colored liquid.

The beads are distributed at the bottom and top of the bottle comprising the composition, their heterogeneous size being from 0.5 to 10 mm.

After moderate manual shaking for 5 seconds, the blue/violet two-colored compositions become readily homogenized as a uniform purply blue solution, and may be vaporized uniformly, using a standard pump-action bottle.

The result on the skin is a fragranced fluid deposit, which feels fresh on application, and after drying has a silky feel on the skin.

Once the bottle has been left to stand, the original two-phase distribution of the composition is regained after 5 minutes.

After two months, the appearance and the organoleptic qualities of composition 2 are unchanged, both at room temperature and at 37°C.

**Claims**

**1.** A composition in the form of an oil-in-water emulsion comprising, in a cosmetically acceptable medium:

(i) a dispersed oily phase comprising at least one apolar hydrocarbon-based oil;
(ii) a continuous aqueous-alcoholic phase comprising at least one $C_1$-$C_4$ monoalcohol;
(iii) at least 1% by weight of fragrancing substance(s), relative to the total weight of the said composition;
(iv) magnesium silicate particles; and
(v) at least one polyalkylene glycol.

**2.** Composition according to Claim 1, **characterized in that** the apolar hydrocarbon-based oil is chosen from linear or branched hydrocarbons of mineral or synthetic origin.

3. The composition according to any one of the preceding claims, **characterized in that** the apolar oil is chosen from a volatile or nonvolatile liquid paraffin oil, hydrogenated isoparaffin, naphthalene oil, a totally or partially hydrogenated liquid polydecene, isoeicosane, a decene/butene copolymer, or a polybutene/polyisobutene copolymer, and mixtures thereof.

4. The composition according to any one of the preceding claims, **characterized in that** said $C_1$-$C_4$ monoalcohol is chosen from linear $C_1$-$C_4$ hydroxyalkyls, advantageously ethanol.

5. Composition according to any one of the preceding claims, **characterized in that** it comprises not more than 30% by weight, in particular from 1% to 25% by weight and especially from 3% to 15% by weight, of fragrancing substance(s) relative to the total weight of the said composition.

6. The composition according to any one of the preceding claims, **characterized in that** said polyalkylene glycol is of formula (I) below:

$$H\text{-}[O\text{-}R\text{-}]_n\text{-}OH \qquad (I),$$

in which

    - R represents a linear alkyl chain containing from 1 to 4 carbon atoms, and
    - n is an integer ranging from 4 to 200, advantageously from 4 to 40.

7. The composition according to any one of the preceding claims, in which the polyalkylene glycol(s) are preferably present in the composition in concentrations ranging from 0.1% to 3% and more preferentially from 0.5% to 2% by weight relative to the total weight of the composition.

8. Composition according to Claim 6 or 7, **characterized in that** n is an integer ranging from 4 to 20, and advantageously from 6 to 10, in the said polyalkylene glycol.

9. Composition according to any one of the preceding claims, **characterized in that** it also comprises at least one water-soluble metal salt preferably chosen from water-soluble alkali metal salts and alkaline-earth metal salts and more preferentially calcium chloride and sodium chloride, and is preferably calcium chloride.

10. The composition according to any one of the preceding claims, **characterized in that** the absolute value of the difference in density ($\Delta d$) between the dispersed oily phase and the continuous aqueous-alcoholic phase is not more than 0.05 and advantageously not more than 0.01.

11. The composition according to any one of the preceding claims, **characterized in that** it comprises at least one liposoluble first colorant in the dispersed oily phase (i) and at least one water-soluble second colorant in the continuous aqueous-alcoholic phase (ii).

12. The composition according to claim 11, **characterized in that** said first and second colorants give the dispersed and continuous phases of said compositions different shades.

13. A process for fragrancing human keratin materials and/or clothing that is in contact with said keratin materials, comprising the application to said keratin materials and/or said clothing of the composition as defined in any one of claims 1 to 12.


**Patentansprüche**

1. Zusammensetzung in Form einer Öl-in-Wasser-Emulsion, umfassend, in einem kosmetisch verträglichen Medium:

    (i) eine disperse ölige Phase umfassend mindestens ein apolares Kohlenwasserstoff basiertes Öl;
    (ii) eine kontinuierliche wässrig-alkoholische Phase umfassend mindestens einen $C_1$-$C_4$-Monoalkohol;
    (iii) mindestens 1 Gew.-% Beduftungssubstanz(en), relativ zum Gesamtgewicht der Zusammensetzung;
    (iv) Magnesiumsilikatteilchen; und
    (v) mindestens ein Polyalkylenglykol.

**2.** Zusammensetzung nach Anspruch 1, **dadurch gekennzeichnet, dass** das apolare Kohlenwasserstoff-basierte Öl aus linearen oder verzweigten Kohlenwasserstoffen mineralischer oder synthetischer Herkunft ausgewählt ist.

**3.** Zusammensetzung nach einem der vorangehenden Ansprüche, **dadurch gekennzeichnet, dass** das apolare Öl aus einem flüchtigen oder nichtflüchtigen flüssigen Paraffinöl, hydrierten Isoparaffin, Naphthalinöl, einem vollständig oder teilweise hydriertem flüssigen Paraffin, Isoeicosan, einem Decen/Buten-Copolymer, oder einem Polybuten/Polyisobuten-Copolymer, und Gemischen davon ausgewählt ist.

**4.** Zusammensetzung nach einem der vorangehenden Ansprüche, **dadurch gekennzeichnet, dass** der $C_1$-$C_4$-Monoalkohol aus linearen $C_1$-$C_4$-Hydroxyalkylen, vorteilhafterweise Ethanol ausgewählt ist.

**5.** Zusammensetzung nach einem der vorangehenden Ansprüche, **dadurch gekennzeichnet, dass** sie nicht mehr als 30 Gew.-%, insbesondere 1 Gew.-% bis 25 Gew.-% und speziell 3 Gew.-% bis 15 Gew.-% Beduftungssubstanz(en), relativ zum Gesamtgewicht der Zusammensetzung umfasst.

**6.** Zusammensetzung nach einem der vorangehenden Ansprüche, **dadurch gekennzeichnet, dass** das Polyalkylenglycol die nachstehende Formel (1) aufweist:

$$H\text{-}[O\text{-}R\text{-}]_n\text{-}OH \qquad (I)$$

wobei

- R eine lineare Alkylkette enthaltend 1 bis 4 Kohlenstoffatome darstellt, und
- n eine ganze Zahl im Bereich von 4 bis 200, vorteilhafterweise von 4 bis 40 ist.

**7.** Zusammensetzung nach einem der vorangehenden Ansprüche, wobei die Polyalkylenglykol(e) vorzugsweise in der Zusammensetzung in Konzentrationen im Bereich von 0,1 Gew.-% bis 3 Gew.-% und stärker bevorzugt von 0,5 bis 2 Gew.-% relativ zum Gesamtgewicht der Zusammensetzung vorhanden sind.

**8.** Zusammensetzung nach Anspruch 6 oder 7, **dadurch gekennzeichnet, dass** n in dem Polyalkylenglycol eine ganze Zahl im Bereich von 4 bis 20, und vorteilhafterweise von 6 bis 10 ist.

**9.** Zusammensetzung nach einem der vorangehenden Ansprüche, **dadurch gekennzeichnet, dass** es auch mindestens ein wasserlösliches Metallsalz umfasst, das vorzugsweise ausgewählt ist aus wasserlöslichen Alkalimetallsalzen und Erdalkalimetallsalzen und stärker bevorzugt aus Calciumchlorid und Natriumchlorid, und das vorzugsweise Calciumchlorid ist.

**10.** Zusammensetzung nach einem der vorangehenden Ansprüche, **dadurch gekennzeichnet, dass** der Absolutwert der Differenz in der Dichte ($\Delta$d) zwischen der dispersen öligen Phase und der kontinuierlichen wässrig-alkoholischen Phase nicht mehr als 0,05 und vorteilhafterweise nicht mehr als 0,01 beträgt.

**11.** Zusammensetzung nach einem der vorangehenden Ansprüche, **dadurch gekennzeichnet, dass** sie mindestens ein fettlösliches erstes Farbmittel in der dispersen öligen Phase (i) und mindestens ein wasserlösliches zweites Farbmittel in der kontinuierlichen wässrig-alkoholischen Phase (i) umfasst.

**12.** Zusammensetzung nach Anspruch 11, **dadurch gekennzeichnet, dass** die ersten und zweiten Farbmittel die disperse und die kontinuierliche Phase der Zusammensetzungen mit verschiedenen Schattierungen ergeben.

**13.** Verfahren zum Beduften von menschlichen keratinischen Materialien und/Bekleidung, die mit den keratinischen Materialien in Kontakt sind, umfassend die Anwendung der Zusammensetzung nach einem der Ansprüche 1 bis 12 auf die keratinischen Materialien und/oder die Bekleidung.

**Revendications**

**1.** Composition sous la forme d'une émulsion huile-dans-eau, comprenant dans un milieu cosmétiquement acceptable

(i) une phase huileuse dispersée comprenant au moins une huile hydrocarbonée apolaire;

(ii) une phase hydroalcoolique continue comprenant au moins un mono-alcool en $C_1$-$C_4$;
(iii) au moins 1 % en poids de substance(s) parfumante(s), par rapport au poids total de ladite composition;
(iv) des particules de silicate de magnésium et
(v) au moins un polyalkylène glycol.

**2.** Composition selon la revendication 1, **caractérisée en ce que** l'huile hydrocarbonée apolaire est choisie parmi les hydrocarbures linéaires ou ramifiés d'origine minérale ou synthétique.

**3.** Composition selon l'une quelconque des revendications précédentes, **caractérisée en ce que** l'huile apolaire est choisie parmi une huile de paraffine liquide, volatile ou non volatile, l'isoparaffine hydrogénée, l'huile de naphtalène, un polydécène liquide totalement ou partiellement hydrogéné, l'isoeicosane, un copolymère décène/butène, ou un copolymère poly-butène/polyisobutène et leurs mélanges.

**4.** Composition selon l'une quelconque des revendications précédentes, **caractérisée en ce que** ledit mono-alcool en $C_1$-$C_4$ est choisi parmi les hydroxyalkyles linéaires en $C_1$-$C_4$, avantageusement l'éthanol.

**5.** Composition selon l'une quelconque des revendications précédentes, **caractérisée en ce qu'**elle comprend au plus 30 % en poids, en particulier de 1 % à 25 % en poids, et notamment de 3 % à 15 % en poids de substance(s) parfumante(s), par rapport au poids total de ladite composition.

**6.** Composition selon l'une quelconque des revendications précédentes, **caractérisée en ce que** ledit polyalkylène glycol est de formule (I) suivante :

$$H\text{-}[O\text{-}R\text{-}]_n\text{-}OH \qquad (I),$$

avec

- R représente une chaîne alkyle linéaire ayant de 1 à 4 atomes de carbone, et
- n est un entier allant de 4 à 200, avantageusement de 4 à 40.

**7.** Composition selon l'une quelconque des revendications précédentes, où le ou les polyalkylène glycol(s) sont de préférence présents dans la composition dans des concentrations allant de 0,1 % à 3 %, et plus préférentiellement de 0,5 % à 2 % en poids par rapport au poids total de la composition.

**8.** Composition selon la revendication 6 ou 7, **caractérisée en ce que** n est un entier allant de 4 à 20 dans ledit polyalkylène glycol, et avantageusement de 6 à 10.

**9.** Composition selon l'une quelconque des revendications précédentes, **caractérisée en ce qu'**elle comprend également au moins un sel métallique hydrosoluble choisi de préférence parmi les sels hydrosolubles de métal alcalin et les sels de métal alcalino-terreux et plus préférentiellement le chlorure de calcium et le chlorure de sodium, et est préférentiellement le chlorure de calcium.

**10.** Composition selon l'une quelconque des revendications précédentes, **caractérisée en ce que** la valeur absolue de la différence de densité ($\Delta$d) entre la phase huileuse dispersée et la phase hydroalcoolique continue est d'au plus 0,05, et avantageusement d'au plus 0,01.

**11.** Composition selon l'une quelconque des revendications précédentes, **caractérisée en ce qu'**elle comprend au moins un premier colorant liposoluble dans la phase huileuse dispersée (i) et au moins un second colorant hydrosoluble dans la phase hydroalcoolique continue (ii).

**12.** Composition selon la revendication 11, **caractérisée en ce que** lesdits premier et second colorants donnent différentes teintes aux phases dispersée et continue desdites compositions.

**13.** Procédé de parfumage des matières kératiniques humaines et/ou d'un vêtement en contact avec lesdites matières kératiniques comprenant l'application sur lesdites matières kératiniques et/ou sur ledit vêtement de la composition telle que définie dans l'une quelconque des revendications 1 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2208642
- EP 1005849 A
- US 4077441 A
- US 4850517 A

**Non-patent literature cited in the description**

- **C.M. HANSEN.** The three dimensional solubility parameters. *J. Paint Technol.,* 1967, vol. 39, 105
- **S. ARCTANDER.** *Perfume and Flavor Chemicals,* 1969
- **S. ARCTANDER.** *Perfume and Flavor Materials of Natural Origin,* 1960
- Flavor and Fragrance Materials. Allured Publishing Co, 1991
- Dictionnaire de la Chimie et de ses Applications. **DUVAL ; DUVAL.** Technique et Documentation. 1978